# EUROPEAN PATENT APPLICATION

(11) **EP 4 606 299 A1**
(43) Date of publication of application: **27.08.2025**
(21) Application number: 24159057.9
(22) Date of filing: 22.02.2024
(51) Int. Cl.: A61B 5/0205, A61B 5/16, A61B 5/00, A61B 5/024

(54) **CALMING A SUBJECT**

(71) Applicant: Koninklijke Philips N.V., 5656 AG Eindhoven (NL)
(72) Inventor: ZHAO, Xinying, Eindhoven (NL); ZHOU, Yi, Eindhoven (NL); YIN, Bin, 5656AG Eindhoven (NL)
(74) Representative: Philips Intellectual Property & Standards

(57) **Abstract**

Proposed concepts thus aim to provide schemes, solutions, concepts, designs, methods and systems pertaining to calming a subject during breathing difficulties. In particular, embodiments aim to provide a method for system for calming a subject during breathing difficulties by generating and outputting an audio signal comprising white noise and a context sound, based on obtained physiological data. In other words, it is proposed that by generating and outputting an audio signal comprising white noise and a context sound, a subject can be calmed using ASMR, therefore breaking them out of the anxiety-breathlessness cycle and/or allowing them to tolerate BiPAP without the need for anxiolytics or sedation.

## Description

### FIELD OF THE INVENTION

This invention relates to the field of breathing difficulties, and more specifically to the field of calming a subject during breathing difficulties.

### BACKGROUND OF THE INVENTION

In patients with respiratory diseases, primarily COPD and asthma, there is a conceptual model that considers the anxiety-breathlessness cycle. It is also called the vicious cycle of flash COPD exacerbation and describes an interplay between emotions and the cardinal symptom of dyspnea. When patients deteriorate, physicians may choose immediate intubation in order to help rest the diaphragm when the patient meets the criteria. However, immediate intubation may not be necessary and therefore avoidable with strategic use of various medication and non-invasive modalities.

The recommended method is to use BiPAP and anxiolytics and monitor the subject in case immediate intubation is needed. Nevertheless, patients may not tolerate BiPAP and may therefore need sedation. In this way, patients may not be awake and therefore not able to provide input for further accurate clinical decision making.

The anxiety-breathlessness cycle starts with disease pathophysiology which leads to dyspnea, which then exacerbates anxiety and emotional responses, which leads to more dyspnea, further exacerbating the emotional dysfunction, comorbid symptoms or behavioral responses (e.g., fatigue and reduced activity). Specifically, when dyspnea happens and is followed by anxiety and tachypnea, the combination of the obstructed airways and tachypnea leads to inadequate expiration time, which causes lung hyperinflation and the diaphragm becoming flattened, making it hard for the patient to inhale. This leads to a worsening disease state, which manifests itself as increased dyspnea, and therefore the anxiety-breathlessness cycle continues. This cycle may cause patients to deteriorate very rapidly but improve rapidly as well.

Normally, the physician will use a combination of BiPAP and anxiolytics to break patients out of this anxiety-breathlessness cycle. If the patient cannot tolerate the BiPAP mask, the physician will typically try some sort of sedation (e.g., dexmedetomidine, low-dose IV haloperidol, benzodiazepines, ketamine, fentanyl, or high-flow nasal cannula). The use of sedation, however, may make the patient unarousable and unable to report how they are feeling.

Typical methods for getting a subject to calm down without sedation, such as paced breathing or progressive muscle relaxation, are unlikely to work for patients with respiratory diseases because patients are already experiencing breathlessness. It is certainly unlikely they would be capable of controlling their breathing rate or any additional physical activity. Furthermore, a reason many patients cannot tolerate the BiPAP mask is because they are too nervous to breathe with a mask providing positive air pressure.

Autonomous sensory meridian response (ASMR) is a scientific term for a perceptual phenomenon characterized by a pleasurable, tingly sensation that begins in the head and scalp and then moves throughout the limbs of the body, causing the subject to relax. It is an involuntary event that occurs when someone is provoked by visual, auditory, olfactory, and/or cognitive stimuli, such as whispering, tapping, or hair brushing. ASMR stimulates the pathways of the brain via frequencies which can trigger the release of hormones, such as dopamine and oxytocin. It is theorized that this is due to high-frequency sounds that act like air conduction on the muscles of the middle ear that regulate three small bones around the eardrum. When the sounds stimulate these muscles, it can slow down the heartbeat and help subjects settle.

### SUMMARY OF THE INVENTION

The invention is defined by the claims.

According to examples in accordance with an aspect of the invention, there is provided a system for calming a subject during breathing difficulties.

The system comprises: a monitor module configured to obtain first physiological data comprising at least one first physiological parameter of the subject indicative of a breathing difficulty of the subject; a process module configured to generate, based on the obtained first physiological data, at least one first audio signal comprising white noise and a context sound; and an audio module configured to output the at least one generated first audio signal.

Proposed concepts thus aim to provide schemes, solutions, concepts, designs, methods and systems pertaining to calming a subject during breathing difficulties. In particular, embodiments aim to provide a system for calming a subject during breathing difficulties by generating and outputting an audio signal comprising white noise and a context sound, based on obtained physiological data.

In other words, it is proposed that by generating and outputting an audio signal comprising white noise and a context sound (e.g., by combining white noise acting as a background sound and a context sound to generate the audio signal), a subject can be calmed using ASMR, therefore breaking them out of the anxiety-breathlessness cycle and/or allowing them to tolerate BiPAP without the need for anxiolytics or sedation. Further, by using less or no sedation, patients can be more conscious and therefore provide more input for clinical decision-making as well as adapting to the BiPAP therapy, if needed, more easily. Hence, in some situations, for patients who can benefit from BiPAP therapy, immediate intubation can be avoided.

By generating the audio signal based on obtained physiological data (comprising one or more physiological parameters of the subject indicative of breathing difficulty), the audio signal can be specifically configured for the current state / situation of the subject. For instance, if the subject is having serious breathing difficulties, more intense white noise can be generated than if the subject is having less serious breathing difficulties and/or the context sound can be changed to be customized to the subject's breathing difficulties. In another example, if the physiological data indicates that the subject needs to try and slow their breathing, the context sound can include a whispered instruction to slow their breathing (e.g., whispered guided breathing).

In other words, by obtaining physiological data and basing a generated audio signal on the physiological data, the audio signal can be automatically tailored / specifically configured to the context of the subject's breathing difficulties. More specifically, the content of the context sound can be specifically configured to the context of the subject's breathing difficulties to comprise, for example, a specific medical instruction, a whisper, a comfort word, etc. The use of physiological data allows the information about the subject's breathing difficulties to be obtained without any subject input.

This invention therefore provides a solution that helps subjects calm down, learn to breathe with a mask and positive air pressure, and/or report their feelings consciously for a physician to decide whether to intubate. By generating an audio signal which utilizes autonomous sensory meridian response (ASMR), the subject can be calmed down and therefore be broken out of the anxiety-breathlessness cycle with no or less medication. The invention can therefore be used to help a patient tolerate a BiPAP mask (or any other potentially stressful device).

This invention would be of particular use in the context of ventilators, both at home and in a clinical setting such as a hospital.

In some embodiments, the context sound may comprise at least one of: a medical instruction; a comfort word; a whisper; a whispered instruction; a whispered comfort word; and a personalized message. These are all sounds which can be useful to provide to a subject to help them calm down by triggering ASMR. In particular, whispers are typically the most effective ASMR triggers.

In some embodiments, the context sound may be at least partially synthesized and may comprise at least one of: a whisper; a whispered instruction; and a whispered comfort word. This allows the whispers to be optimized to induce ASMR in a subject by, for example, standardizing the frequencies present in the whisper.

In some embodiments, the semantic content of the context sound may be associated with a stage in a clinical treatment phase of the subject. This allows the generated audio signal to be more relevant to the subject by including, for example, instructions associated with a present stage of a clinical treatment of the subject.

In some embodiments, the process module may be further configured to: analyze the obtained first physiological data to determine at least one of: an anxiety breathlessness score; a bilevel positive airway pressure (BiPAP) tolerance score; and an indication of intubation; wherein generating the at least one first audio signal may be based on the at least one of the anxiety breathlessness score; the BiPAP tolerance score; and the indication of intubation indicating a presence of breathing difficulties of the subject. In other words, if any of the anxiety breathlessness score, BiPAP tolerance score, and/or indication of intubation indicate that the subject is having breathing difficulties, then the audio signal can be generated based on the relevant score(s) and/or indications.

In some embodiments, after outputting the at least one generated first audio signal, the monitor module may be configured to obtain second physiological data comprising at least one second physiological parameter of the subject indicative of a breathing difficulty of the subject. This may allow the effectiveness of the output first audio signal to be determined and/or a clinical trajectory of the subject to be tracked.

In some embodiments, if the first physiological data and the second physiological data indicates that the subject's breathing difficulty is stable or improving, the process module may be configured to: generate at least one second audio signal comprising the same white noise as the first audio signal and a different context sound to the first audio signal. For example, if the subject is stable or getting better, there may be no need to change the white noise, which is likely to be effective, however, the context sound may be changed to let the subject know that they are getting better or to provide them with new instructions or words of encouragement, for example.

In some embodiments, if the first physiological data and the second physiological data indicates that the subject's breathing difficulty is getting worse, the process module may be configured to: generate at least one second audio signal comprising at least one of: a different white noise to the first audio signal and the same context sound as the first audio signal; the same white noise as the first audio signal and a different context sound to the first audio signal; and a different white noise and a different context sound to the first audio signal. This facilitates one or both aspects of the audio signal being adjusted in response to the first audio signal not improving the breathing difficulties of the subject. This cycle may then repeat until a combination is found that effectively calms the subject and therefore improves their breathing difficulties.

In some embodiments, the at least one second physiological parameter may comprise the same parameter as the at least one first physiological parameter, and an indication that the subject's breathing difficulty is stable, improving or getting worse may be based on a change between the at least one second and at least one first physiological parameter. This provides an effective and/or efficient way to determine the trajectory of the subject's breathing difficulties.

In some embodiments, the system may further comprise a feedback module configured to: determine a clinical trend based on the first physiological data and the second physiological data; determine a calming effectiveness score of the at least one first audio signal based on the determined clinical trend; and generate a display control signal describing the clinical trend and the calming effectiveness score. In this way, a clinician may be efficiently and/or effectively provided with an overview of the subject's current state and the effectiveness of the provided first audio signal in calming the subject.

In some embodiments, the feedback module may be further configured to display a predetermined number of combinations of different white noises and different context sounds, which has the best effectiveness (e.g., in a past predetermined time period).

In some embodiments, the feedback module may be further configured to receive user input; and wherein the process module may be further configured to generate at least one further audio signal based on the received user input. In this way, a user (either the subject or a clinician) may influence the content of the audio signal, for instance, to change the type of white noise or to change the context sound.

In some embodiments, the first physiological data may comprise at least one physiological parameter of the subject describing a stress level of the subject and at least one physiological parameter of the subject describing respiration of the subject. Having the physiological data comprise parameters describing both the stress level and respiration of the subject ensures a fuller picture of the subject's clinical status is provided.

In some embodiments, the at least one first physiological parameter may comprise at least one of: respiratory rate; blood oxygen level; tidal volume of the subject; minute ventilation; heart rate variability; skin resistance level; subject-ventilator asynchrony; FiO2; oxygen saturation; and work of breathing. These are all useful physiological parameters to obtain in order to indicate breathing difficulties.

In some embodiments, the audio module may comprise a speaker configured to play the at least one output first audio signal. This is an effective way to provide the subject with the generated audio signal.

In some embodiments, the process module may be configured to test different combinations of different white noises and different context sounds, each for a predetermined period of time, and to select the combination which results in the best calming effectiveness score (i.e., which results in the subject's breathing difficulties improving the most or, if none improve the subject's breathing difficulties, which results in the subject's breathing difficulties getting the least worse).

According to another aspect of the invention, there is provided a ventilator comprising: a breathable gas source; a delivery system for delivering a breathable gas from the breathable gas source to the subject, the delivery of the breathable gas being directed to the subject through a respiratory conduit of the ventilator; and the system of any above-described embodiment.

According to another aspect of the invention, there is provided a computer program comprising code means for implementing the following method when said program is run on a system according to any above-described embodiment: obtaining first physiological data comprising at least one first physiological parameter of the subject indicative of a breathing difficulty of the subject; generating, based on the obtained first physiological data, at least one first audio signal comprising white noise and a context sound; and outputting the at least one generated first audio signal

Thus, there may be proposed concepts for calming a subject during breathing difficulties, and this may be done based on generating and outputting an audio signal comprising white noise and a context sound, based on obtained physiological data.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 is a simplified block diagram of a system for calming a subject during breathing difficulties according to a proposed embodiment;
Fig. 2 is a flow diagram showing a process for generating a second audio signal using a system according to a proposed embodiment;
Fig. 3 is a block diagram of a system for calming a subject during breathing difficulties according to a proposed embodiment;
Fig. 4 is a simplified block diagram of a ventilator according to a proposed embodiment;
Fig. 5 is a flow diagram of a method implemented by code means according to a proposed embodiment; and
Fig. 6 illustrates an example of a computer within which one or more parts of an embodiment may be employed.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

Implementations in accordance with the present disclosure relate to various techniques, methods, schemes and/or solutions pertaining to calming a subject during breathing difficulties. According to proposed concepts, a number of possible solutions may be implemented separately or jointly. That is, although these possible solutions may be described below separately, two or more of these possible solutions may be implemented in one combination or another.

Embodiments of the invention aim to provide a system for calming a subject during breathing difficulties. Proposed concepts thus aim to provide schemes, solutions, concepts, designs, methods and systems pertaining to calming a subject during breathing difficulties. In particular, embodiments aim to provide a system for calming a subject during breathing difficulties by generating and outputting an audio signal comprising white noise and a context sound, based on obtained physiological data.

In other words, it is proposed that by generating and outputting an audio signal comprising white noise and a context sound, a subject can be calmed using ASMR, therefore breaking them out of the anxiety-breathlessness cycle and/or allowing them to tolerate BiPAP without the need for anxiolytics or sedation. Further, by using less or no sedation, patients can be more conscious and therefore provide more input for clinical decision making as well as adapting to the BiPAP therapy, if needed, more easily. Hence, in some situations, for patients who can benefit from BiPAP therapy, immediate intubation can be avoided.

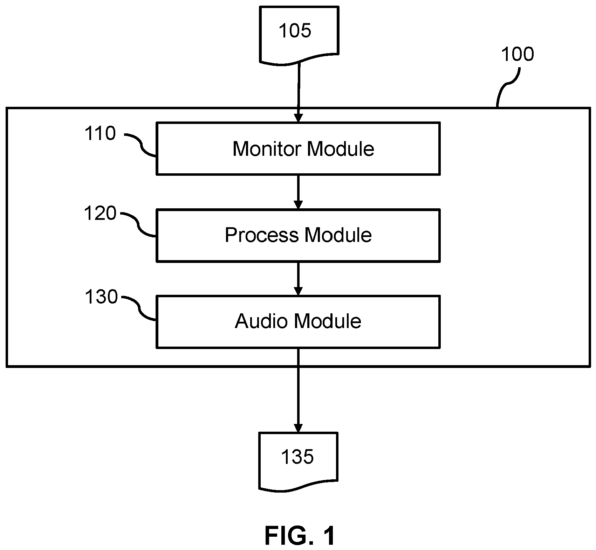

Referring now to Fig. 1, there is depicted a simplified block diagram of a system 100 for calming a subject during breathing difficulties according to a proposed embodiment.

The system 100 comprises a monitor module 110, a process module 120, and an audio module 130. The monitor module 110 is configured to obtain first physiological data 105 comprising at least one first physiological parameter of the subject indicative of a breathing difficulty of the subject.

In this embodiment, the at least one first physiological parameter comprises at least one of: respiratory rate; blood oxygen level; tidal volume of the subject; minute ventilation; heart rate variability; skin resistance level; subject-ventilator asynchrony; FiO2; oxygen saturation; and work of breathing. These are all useful physiological parameters to obtain in order to indicate breathing difficulties, however, in other embodiments, the first physiological parameter can comprise any suitable parameter which indicates breathing difficulties of a subject. For example, it has been found particularly beneficial for the first physiological parameters to comprise respiratory rate; blood oxygen level; and tidal volume of the subject.

Respiratory rate describes the number of breaths taken per minute, reflecting the frequency of respiratory cycles, while blood oxygen level indicates the concentration of oxygen in the bloodstream essential for cellular function. The tidal volume of the subject represents the volume of air exchanged during a normal breath, and minute ventilation quantifies the total air moved in and out of the lungs per minute, determined by the product of respiratory rate and tidal volume. Heart rate variability signifies the fluctuations in time intervals between successive heartbeats. Skin resistance level measures the electrical resistance of the skin, influenced by sympathetic nervous system activity and emotional responses. Subject-ventilator asynchrony refers to the lack of coordination between mechanical ventilation and the patient's respiratory efforts. FiO2 represents the fraction of inspired oxygen in the air, expressed as a percentage, while oxygen saturation reflects the percentage of hemoglobin binding sites occupied by oxygen molecules in the bloodstream. Lastly, the work of breathing characterizes the energy expended by respiratory muscles to move air in and out of the lungs, indicating the effort required for breathing.

In some embodiments, the first physiological data comprises at least one physiological parameter of the subject describing a stress level of the subject and at least one physiological parameter of the subject describing respiration of the subject. Having the physiological data comprise parameters describing both the stress level and respiration of the subject ensures a fuller picture of the subject's clinical status is provided.

For example, the monitor module 110 can monitor a subject's anxiety level through any accessible methodology or combined methodology, e.g., a subject's report based on an anxiety scale, heart rate variability (HRV), or skin resistance level, etc. The subject's respiratory condition can also be monitored through any accessible dataset from any suitable medical or non-medical devices or any algorithm for assessing the subject's respiratory condition, e.g., respiratory rate, blood oxygen level, tidal volume, minute ventilation, ABG/VBG results, etc. For example, if the subject's anxiety level and respiratory condition are significantly correlated (e.g., r > 0.2, p < 0.05) and the deterioration of the subject's respiratory condition is significant in a short period (e.g., 3 minutes), then this can indicate the subject is entering an anxiety-breathlessness cycle. In some embodiments, this can be detected by the process module 120).

The process module 120 is configured to generate, based on the obtained first physiological data, at least one first audio signal comprising white noise and a context sound.

White noise, a sound characterized by a consistent distribution of frequencies with equal intensity, can take various forms to create a soothing background ambiance. Examples include the static noise of a television without a signal, the continuous crackling of radio static, the rhythmic crashing of ocean waves, the steady patter of rainfall, the rustling of wind through trees, the hum of a fan or air conditioner, the flowing water of a river or waterfall, the consistent background noise in an airplane cabin, the soft humming of electrical appliances, and even the neutral combination of tones in soft music or instrumental tracks.

In other words, white noise is a collection of sound vibrations that contain all known frequencies and so can be used to mask other sounds and thereby give listeners something upon which to concentrate. White noise masks ambient noise and so can be used as a background sound over which to overlay the context sound, thereby allowing the user to more clearly focus on the context sound.

In this embodiment, the context sound comprises at least one of: a medical instruction; a comfort word; a whisper; a whispered instruction; a whispered comfort word; and a personalized message. These are all sounds which can be useful to provide to a subject to help them calm down by triggering ASMR. In particular, whispers are typically the most effective ASMR triggers. In other embodiments, however, the context sound can comprise any suitable sound for triggering ASMR, as would be understood by the skilled person.

It should be noted a context sound can also be referred to as a content sound or an ASMR-triggering sound.

For example, in some embodiments, the system 100 can further comprise an audio input device to allow context sounds to be input into the system 100. For example, the audio input device can comprise any hardware with audio recording capability, such as a microphone.

In fact, in this embodiment, the context sound is at least partially synthesized and comprises at least one of: a whisper; a whispered instruction; and a whispered comfort word. This allows the whispers to be optimized to induce ASMR in a subject by, for example, standardizing the frequencies present in the whisper. For example, a voice changer could be used to achieve more ideal ASMR-triggering audio signals. Again, it should be noted that this is not essential to the working of the invention and in other embodiments, the context sound can comprise any suitable sound for triggering ASMR. For example, other sounds that can trigger ASMR comprise: tapping; crinkling; brushing sounds; soft speech; gentle eating or drinking; page-turning; purring; gentle animal sounds; hair brushing sounds; dripping water; fabric rustling; typing on a keyboard; pencil or pen writing sounds; a lid opening and closing.

In this embodiment, the semantic content of the context sound is also associated with a stage in a clinical treatment phase of the subject. This allows the generated audio signal to be more relevant to the subject by including, for example, instructions, guidance and/or a description associated with a present stage of a clinical treatment of the subject. It should be noted that this feature is not essential to the working of the invention, as would be understood by the skilled person.

In other words, the content of the context sound can be different depending on the different current stages in the clinical phase. For example, some phases could be:
1st phase: Just entered the anxiety-dyspnea cycle and no treatment has been implemented;
2nd phase: Non-invasive ventilation therapy/high-flow oxygen has been implemented, but the patient has tolerance problems, treatment could help but treatment burden is high; and
3rd phase: Non-invasive ventilation therapy/high-flow oxygen has been implemented but is ineffective and invasive ventilation therapy is required.

For example, for a subject in the 1st phase, the context sound could comprise: an explanation of the patient's symptoms, empathy, reassurance, encouragement (for the respiratory-anxiety cycle); a description of the treatment that the physician may want to perform (the patient will try breathing technique training first, and may need to undergo non-invasive ventilation/oxygen therapy if still feeling severely dyspneic), and what needs to be done with the patient's cooperation (non-invasive ventilation may require wearing a mask, and oxygen therapy will require wearing a nasal mask); and/or breathing techniques (abdominal breathing, lip-contraction breathing) that the patient can practice on his/her own.

For example, for a subject in the 2nd phase, the context sound can comprise: an explanation of patient's symptoms, empathy, reassurance, encouragement (for tolerance problems, e.g., asynchrony of the ventilator, mask pressure, dry mouth, etc.); an introduction of possible treatments to be carried out by the doctor, what needs to be done by the patient, e.g. the patient may face ventilator tolerance problems and how to cope with them (e.g. call the nurse to help hydrate the patient if he/she has a dry mouth); and/or breathing techniques that the patient can practice on his/her own (e.g. how to breathe to adapt to the ventilator and how to synchronize with the ventilator).

For example, for a subject in the 3rd phase, the context sound can comprise: an explanation of the patient's symptoms, empathy, reassurance, and encouragement (in response to the patient's severe non-invasive ventilator tolerance issues and the severity of the current dyspnea); an introduction of the treatment that the doctor may need to carry out (tracheal intubation process, informed consent, etc.), and what needs to be done by the patient (the doctor will elevate the bed by 30-45 degrees to reduce the risk of aspiration, and the patient needs to cooperate with the patient to avoid lying down as much as possible, if the patient is unable to speak, he/she can communicate with the healthcare personnel through eye gestures, etc. to express his/her needs); and/or breathing techniques that the patient can practice on his/her own (e.g., keep quiet and do not breathe strenuously; try deep breathing to improve lung function).

For each phase above, a default priority can be to have the context sound comprise a medical professional's whispered content, followed by a family member's whispered content, followed by further relevant content according to the current phase.

For example, in this invention, the generated audio signal is intended to trigger ASMR and refers to an audio signal comprising a context sound (for example, prerecorded and potentially recorded by a clinician and/or one of the subject's relatives/friends) plus white noise, which acts as a background sound. The ASMR audio recordings can be generated and composed with a source of ASMR audio materials. For example, they can be white noise (such as a static television, the sound of waves, a bell, etc.) and context sounds such as a (whispered) BiPAP therapy introduction for the subject. The introduction, for example, could comprise a physician's introduction to the subject's upcoming non-invasive ventilation treatment, such as the purpose of the treatment, what is required of the subject, what the subject may experience, how he or she needs to cope, and other educational information. The recording could preferably be given in the form of a whisper. Other kinds of material could also be used, for example, if the patient's condition is severe or deteriorating, such as comfort words, again, preferably whispered. If visitation is not allowed, recordings from subject's families or friends could also be used. For example, the content type can include: explanation of the patient's symptoms, empathy, reassurance, encouragement; a description of the treatment that the physician is about to perform, what needs to be done by the subject; ventilator tolerance issues that the patient may face and how to deal with them; breathing techniques that the patient can practice on his/her own.

In some embodiments, the context sound can be live recorded by a healthcare professional, for example, such that they are talking into a microphone, and the subject can hear the live context sound through headphones, for example, over white noise.

In some embodiments, the process module 120 can be further configured to: analyze the obtained first physiological data to determine at least one of: an anxiety breathlessness score; a bilevel positive airway pressure (BiPAP) tolerance score; and an indication of intubation; wherein generating the at least one first audio signal may be based on the at least one of the anxiety breathlessness score; BiPAP tolerance score; and/or indication of intubation indicating a presence of breathing difficulties of the subject. In other words, if any of the anxiety breathlessness score, BiPAP tolerance score, and/or indication of intubation indicate that the subject is having breathing difficulties, then the audio signal can be generated based on the relevant score(s). The generated first audio signal can thus comprise different context sounds and/or white noise depending on which score has been calculated and/or is indicating breathing difficulties. For instance, a calculated anxiety breathlessness score being above a predetermined threshold can trigger the generation of a first audio signal comprising a particular context sound; a calculated BiPAP tolerance score being below a different predetermined threshold can trigger the generation of a first audio signal comprising a different particular context sound; and a calculated indication of intubation being positive can trigger the generation of a first audio signal comprising yet a different particular context sound.

Of course, it would be obvious to the skilled person that in embodiments wherein a BiPAP tolerance score is being determined, the subject must be undergoing BiPAP.

In other words, in some embodiments, the process module 120 can be configured to generate at least one first audio signal if any combination of the following three events are detected: anxiety-breathlessness cycle; BiPAP tolerance problems; and/or indication of intubations. This is because it can be uncertain what therapeutic state a subject is in when they enter a therapeutic environment. For example, they may have just entered the anxiety-dyspnea cycle without any treatment being implemented; non-invasive ventilation may have been administered but they have tolerance problems; or invasive ventilation is needed.

Many physiological parameters can be used to indicate need to intubation. For example, oxygen saturation, FiO2, respiratory rate, work of breathing, tidal volume, minute ventilation, ABG/VBG results and mental status.

In this embodiment, the generated first audio signal has a duration between one and two minutes, but in other embodiments, the audio signal can have any suitable duration, for example, between thirty seconds and three minutes or between ten seconds and five minutes.

The audio module 130 is configured to output the at least one generated first audio signal 135. For example, the audio signal can be output to a speaker or a pair of headphones/earphones. For instance, in some embodiments, the audio module 130 can comprise a speaker configured to play the at least one output first audio signal. In other embodiments, however, the audio module 130 can comprise any suitable component for playing or outputting the first audio signal.

For example, the audio module 130 can be configured to output the at least one generated first audio signal 135 when it is determined that anxiety-breathlessness is getting worse (and/or is already bad enough), BiPAP tolerance condition is getting worse (and/or is already bad enough), and/or there are indications of (immediate) intubation.

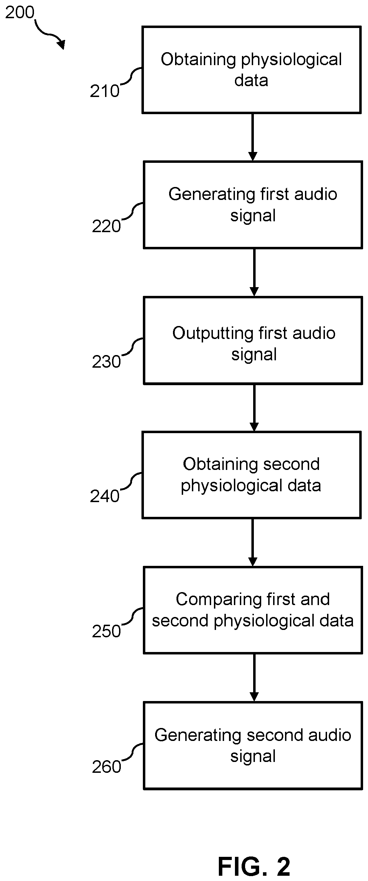

Referring now to Fig. 2, there is depicted a flow diagram showing a process (method) 200 for generating a second audio signal using a system according to a proposed embodiment, such as the system 100 depicted in Fig. 1.

For instance, the monitor module 110 is configured to perform step 210 of obtaining first physiological data comprising at least one first physiological parameter of the subject indicative of a breathing difficulty of the subject. The process module 120 is configured to perform step 220 of generating, based on the obtained first physiological data, at least one first audio signal comprising white noise and a context sound. The audio module 130 is configured to perform step 230 of outputting the at least one generated first audio signal.

In this embodiment, after the audio module 130 outputs the at least one generated first audio signal (step 230), the monitor module 110 is configured to obtain second physiological data comprising at least one second physiological parameter of the subject indicative of a breathing difficulty of the subject (step 240). This allows the effectiveness of the output first audio signal to be determined and/or a clinical trajectory of the subject to be tracked. As should be clear to the skilled person, this feature is not essential to the working of the invention. It should also be noted that in this embodiment, the second physiological data is captured after the at least one generated first audio signal has been output.

In step 250, the first and second physiological data are compared, for instance, by the process module 120. For example, in this embodiment, if the first physiological data and the second physiological data indicates that the subject's breathing difficulty is stable or improving, the process module 120 is configured to: generate at least one second audio signal (step 260) comprising the same white noise as the first audio signal and a different context sound to the first audio signal. For example, if the subject is stable or getting better, there is likely no need to change the white noise, which is likely to be effective, however, the context sound can be changed to let the subject know that they are getting better or to provide them with new instructions or words of encouragement, for example.

In another example, if the first physiological data and the second physiological data indicates that the subject's breathing difficulty is getting worse, the process module 120 is configured to: generate at least one second audio signal (step 260) comprising at least one of: a different white noise to the first audio signal and the same context sound as the first audio signal; the same white noise as the first audio signal and a different context sound to the first audio signal; and a different white noise and a different context sound to the first audio signal. This facilitates one or both aspects of the first audio signal being adjusted in response to the first audio signal not improving the breathing difficulties of the subject. This cycle can then repeat until a combination is found that effectively calms the subject and therefore improves their breathing difficulties. For example, each combination can be tested once or, for example, the best combination can simply be used after testing combinations for a total of 30 minutes.

In this embodiment, the at least one second physiological parameter comprises the same parameter as the at least one first physiological parameter, and an indication that the subject's breathing difficulty is stable, improving or getting worse is based on a change between the at least one second and at least one first physiological parameter (i.e., in step 250). This can provide an effective and/or efficient way to determine the trajectory of the subject's breathing difficulties.

In an example, if the patient's situation is better or stable over, for example, a 3-minute duration, then positive feedback (e.g., "Good! You are doing a great job! ") and guidance to follow (e.g., "Just relax and tell me how you are feeling") and/or breathing techniques can be given along with the same white noise. In other words, the content in the context sounds can progress while the effective white noise can be kept the same.

In another example, if the patient's situation is getting worse over a predetermined duration (e.g., 3-minute duration), then warnings / alerts can be given to healthcare professionals for necessary measurement and treatment (e.g., non-invasive ventilation, anxiolytics), and/or another audio signal can be generated which includes a change in terms of the context sound and/or the background white noise. For example, the background white noise could change to another type in a predefined time period (3 minutes, for example) while the context sound is kept the same. The background noise could keep changing until one is found which helps the subject's breathing difficulties improve within a predefined time period. For example, the white noise could be kept unchanged, while the context sound is changed to another sound suitable for the present state of the subject and/or phase of the clinical treatment. Again, the context sound could keep changing until one is found which most improves the subject's breathing difficulties. For example, both the background white noise and the context sound could change and keep changing until a combination which most helps the subject is found. In all the above examples, if the subject is stabilized and needs to rest, there may be periods of silence (i.e., no audio signal being output) between the generated audio signals.

For example, in some embodiments, the test time for each combination of white noise and context sound is no less than a predetermined duration (e.g., three minutes, or a longer predetermined duration, five minutes, for example, if a heart-related variability indicator is being used for the subject's physiological data). If after all combinations have been tested once (or after a combined test time of, for example, thirty minutes), if the patient is still deteriorating (i.e., not stabilizing or improving), then audio signals can stop being generated and BiPAP and/or anxiolytics may need to be used.

It should be noted that trying a combination for three minutes is merely provided as an example and, as the skilled person would understand, can change depending on the sampling rate of the relevant device for obtaining physiological parameters. However, preferably, the test time should be no less than three minutes, and no less than five minutes if a HRV parameter is being used.

For example, if the subject undergoes or is undergoing BiPAP, then the content of the generated audio signal can accordingly be changed. For example, the context sound can comprise an introduction of BiPAP therapy, how to adapt to the mask, what to expect, and what side effects might be, etc. These context sounds can be arranged in a loop such that the subject is continually reminded and calmed by them. The background white noise can be kept the same as the most effective white noise from before BiPAP (if audio signals were being output before BiPAP) or alternatively, different white noises can be tested until the most effective is found.

For example, even after the best combination of white noise and context sound has been found, the present invention can occasionally test out a different combination of white noise and context sound in case the subject's status has changed (and so accordingly they may react differently to the combinations than they did before).

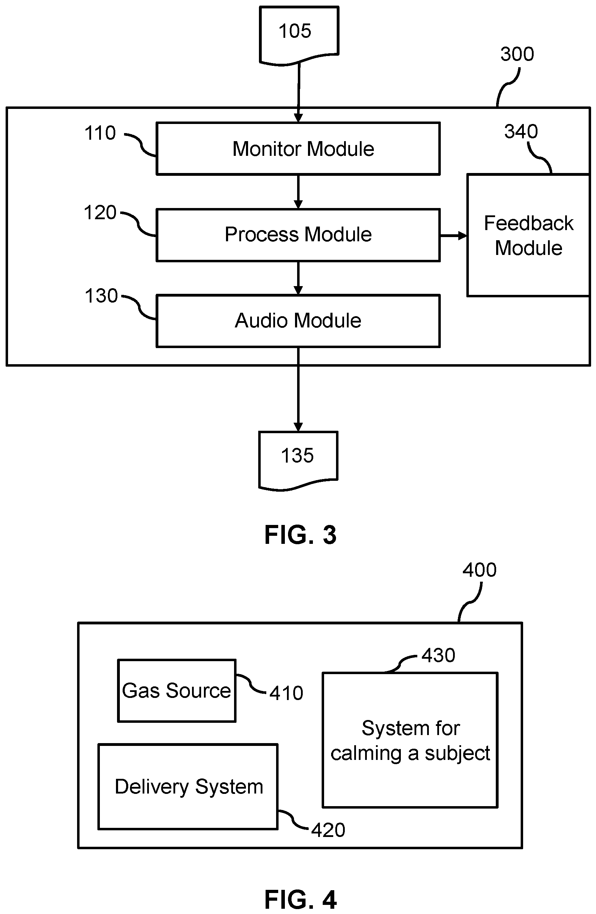

Referring now to Fig. 3, there is depicted a block diagram of a system 300 for calming a subject during breathing difficulties according to a proposed embodiment. The system 300 is essentially the same as system 100 depicted in Fig. 1, however, further including a feedback module 340.

The feedback module 340 is configured to determine a clinical trend based on the first physiological data and the second physiological data; determine a calming effectiveness score of the at least one first audio signal based on the determined clinical trend; and generate a display control signal describing the clinical trend and the calming effectiveness score. In this way, a clinician can be efficiently and/or effectively provided with an overview of the subject's current state and the effectiveness of the provided first audio signal in calming the subject.

In this embodiment, the feedback module 340 is further configured to receive user input; and wherein the process module 120 is further configured to generate at least one further audio signal based on the received user input. In this way, a user (either the subject or a clinician) can influence the content of the audio signal, for instance, to change the type of white noise or to change the context sound.

In an example, the feedback module 340 can display feedback for clinicians based on the physiological parameters (e.g., indicating a patient's overall status, indications which have deviated from a normal range, etc.) and can allow control of context sounds and white noise (either selecting or turning on or off, for example).

For example, the feedback module can show health care professionals the clinical trend of the subject's condition, and whether the generated audio signal(s) have had an effect on the subject's condition in a time period of, for example, 15-30 minutes. Trends in the subject's condition (measured using the physiological data) can be classified as stable, better, or worse. If the physician is just making a simple judgement from a data graph, they can count the subject's physiological data (such as, respiratory rate, SP02, work of breath, etc.) during the period the audio signal was being output and then can manually or automatically plot a fitted curve to determine whether the trend is stable, getting better or getting worse.

For example, four methods can be used separately or in combination to determine the effectiveness of the output audio signal (and can be performed by the feedback module 140). First, a correlation test can be used. This comprises, for example, obtaining a trend for the obtained physiological data, and then determining a relationship between the output audio signal and the patient's trend. Second, the magnitude of the variance value of changes in the physiological data can be evaluated by calculating the variance of the relevant physiological data over the time period that the audio signal was being output and then by comparing this variance to the variance over the time period when the audio signal was not being output to see if the physiological data was stabilized as a result of the ASMR stimulation. Third, the overall trend fit of the physiological data could be determined to essentially visualize the physiological data according to various time scales (second, minute, hour, etc.) using the calculation of mean and standard deviation during a time window. This can allow healthcare professionals to read the physiological data more easily to draw their own conclusions as to the effectiveness of the output audio signal. Fourth, causality tests for time-series data can be used. For example, time-lag analysis can be applied to the physiological data and a regression model can be built to test the relationship between the output audio signal in T and the physiological data in T+1. This allows any significant positive or negative relationship between them to be determined. An analysis of time-series data can be performed to assess the effect on the physiological data after a small period of time after the stimulation was administered. The causality test is the most rigorous method of determining the effect of the audio signal.

For example, the feedback module can also display the current audio signal settings of both the context sound and the choice of white noise. For example, the feedback module can calculate the effectiveness of each combination of context sound and white noise and then display the top three effective combinations. In this way, the subject or a healthcare professional can change between the top three combinations, if for example, the subject gets bored of listening to the same audio signal. In one embodiment, patients can also request to change the combination when the patient does not feel comfortable, boring on the current combination. In this case, as described previously, the feedback module can be further configured to receive user input, and the process module can be further configured to generate at least one further audio signal based on the received user input.

Referring now to Fig. 4, there is depicted a simplified block diagram of a ventilator 400 according to a proposed embodiment. The ventilator 400 comprises a breathable gas source 410; a delivery system 420 for delivering a breathable gas from the gas source 410 to the subject, the delivery of the breathable gas being directed to the subject through a respiratory conduit of the ventilator; and the system 430 of any herein described embodiment (such as system 100 or 300, for example).

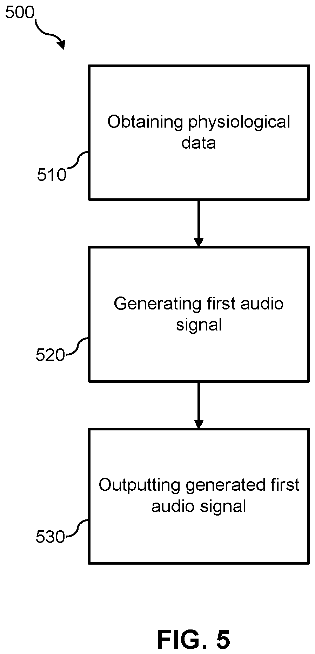

Referring now to Fig. 5, there is depicted a flow diagram of a method 500 implemented by code means according to a proposed embodiment. For instance, there is provided a computer program comprising code means for implementing the following method 500 when said program is run on a system according to any above-described embodiment: obtaining first physiological data comprising at least one first physiological parameter of the subject indicative of a breathing difficulty of the subject (step 510); generating, based on the obtained first physiological data, at least one first audio signal comprising white noise and a context sound (step 520); and outputting the at least one generated first audio signal (step 530).

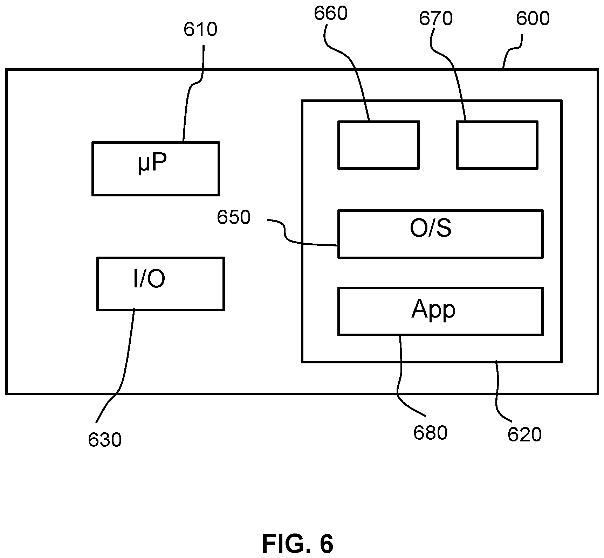

Fig. 6 illustrates an example of a computer 600 within which one or more parts of an embodiment may be employed. Various operations discussed above may utilize the capabilities of the computer 600. In this regard, it is to be understood that system functional blocks can run on a single computer or may be distributed over several computers and locations (e.g. connected via internet).

The computer 600 includes, but is not limited to, PCs, workstations, laptops, PDAs, palm devices, servers, storages, and the like. Generally, in terms of hardware architecture, the computer 600 may include one or more processors 610, memory 620 and one or more I/O devices 630 that are communicatively coupled via a local interface (not shown). The local interface can be, for example but not limited to, one or more buses or other wired or wireless connections, as is known in the art. The local interface may have additional elements, such as controllers, buffers (caches), drivers, repeaters, and receivers, to enable communications. Further, the local interface may include address, control, and/or data connections to enable appropriate communications among the aforementioned components.

The processor 610 is a hardware device for executing software that can be stored in the memory 620. The processor 610 can be virtually any custom made or commercially available processor, a central processing unit (CPU), a digital signal processor (DSP), or an auxiliary processor among several processors associated with the computer 600, and the processor 610 may be a semiconductor based microprocessor (in the form of a microchip) or a microprocessor.

The memory 620 can include any one or combination of volatile memory elements (e.g., random access memory (RAM), such as dynamic random access memory (DRAM), static random access memory (SRAM), etc.) and non-volatile memory elements (e.g., ROM, erasable programmable read only memory (EPROM), electronically erasable programmable read only memory (EEPROM), programmable read only memory (PROM), tape, compact disc read only memory (CD-ROM), disk, diskette, cartridge, cassette or the like, etc.). Moreover, the memory 620 may incorporate electronic, magnetic, optical, and/or other types of storage media. Note that the memory 620 can have a distributed architecture, where various components are situated remote from one another, but can be accessed by the processor 610.

The software in the memory 620 may include one or more separate programs, each of which comprises an ordered listing of executable instructions for implementing logical functions. The software in the memory 620 includes a suitable operating system (O/S) 650, compiler 660, source code 670, and one or more applications 680 in accordance with exemplary embodiments. As illustrated, the application 680 comprises numerous functional components for implementing the features and operations of the exemplary embodiments. The application 680 of the computer 600 may represent various applications, computational units, logic, functional units, processes, operations, virtual entities, and/or modules in accordance with exemplary embodiments, but the application 680 is not meant to be a limitation.

The operating system 650 controls the execution of other computer programs, and provides scheduling, input-output control, file and data management, memory management, and communication control and related services. It is contemplated by the inventors that the application 680 for implementing exemplary embodiments may be applicable on all commercially available operating systems.

Application 680 may be a source program, executable program (object code), script, or any other entity comprising a set of instructions to be performed. When a source program, then the program is usually translated via a compiler (such as the compiler 660), assembler, interpreter, or the like, which may or may not be included within the memory 620, so as to operate properly in connection with the O/S 650. Furthermore, the application 680 can be written as an object oriented programming language, which has classes of data and methods, or a procedure programming language, which has routines, subroutines, and/or functions, for example but not limited to, C, C++, C#, Pascal, Python, BASIC, API calls, HTML, XHTML, XML, ASP scripts, JavaScript, FORTRAN, COBOL, Perl, Java, ADA, .NET, and the like.

The I/O devices 630 may include input devices such as, for example but not limited to, a mouse, keyboard, scanner, microphone, camera, etc. Furthermore, the I/O devices 630 may also include output devices, for example but not limited to a printer, display, etc. Finally, the I/O devices 630 may further include devices that communicate both inputs and outputs, for instance but not limited to, a NIC or modulator/demodulator (for accessing remote devices, other files, devices, systems, or a network), a radio frequency (RF) or other transceiver, a telephonic interface, a bridge, a router, etc. The I/O devices 630 also include components for communicating over various networks, such as the Internet or intranet.

If the computer 600 is a PC, workstation, intelligent device or the like, the software in the memory 620 may further include a basic input output system (BIOS) (omitted for simplicity). The BIOS is a set of essential software routines that initialize and test hardware at start-up, start the O/S 650, and support the transfer of data among the hardware devices. The BIOS is stored in some type of read-only-memory, such as ROM, PROM, EPROM, EEPROM or the like, so that the BIOS can be executed when the computer 600 is activated.

When the computer 600 is in operation, the processor 610 is configured to execute software stored within the memory 620, to communicate data to and from the memory 620, and to generally control operations of the computer 600 pursuant to the software. The application 680 and the O/S 650 are read, in whole or in part, by the processor 610, perhaps buffered within the processor 610, and then executed.

When the application 680 is implemented in software it should be noted that the application 680 can be stored on virtually any computer readable medium for use by or in connection with any computer related system or method. In the context of this document, a computer readable medium may be an electronic, magnetic, optical, or other physical device or means that can contain or store a computer program for use by or in connection with a computer related system or method.

The application 680 can be embodied in any computer-readable medium for use by or in connection with an instruction execution system, apparatus, or device, such as a computer-based system, processor-containing system, or other system that can fetch the instructions from the instruction execution system, apparatus, or device and execute the instructions. In the context of this document, a "computer-readable medium" can be any means that can store, communicate, propagate, or transport the program for use by or in connection with the instruction execution system, apparatus, or device. The computer readable medium can be, for example but not limited to, an electronic, magnetic, optical, electromagnetic, infrared, or semiconductor system, apparatus, device, or propagation medium.

The systems of Figs. 1, 3 and 4, and the method of Figs. 2 and 5, may be implemented in hardware or software, or a mixture of both (for example, as firmware running on a hardware device). To the extent that an embodiment is implemented partly or wholly in software, the functional steps illustrated in the process flowcharts may be performed by suitably programmed physical computing devices, such as one or more central processing units (CPUs) or graphics processing units (GPUs). Each process - and its individual component steps as illustrated in the flowcharts - may be performed by the same or different computing devices. According to embodiments, a computer-readable storage medium stores a computer program comprising computer program code configured to cause one or more physical computing devices to carry out an encoding or decoding method as described above when the program is run on the one or more physical computing devices.

Storage media may include volatile and non-volatile computer memory such as RAM, PROM, EPROM, and EEPROM, optical discs (like CD, DVD, BD), magnetic storage media (like hard discs and tapes). Various storage media may be fixed within a computing device or may be transportable, such that the one or more programs stored thereon can be loaded into a processor.

To the extent that an embodiment is implemented partly or wholly in hardware, the blocks shown in the block diagrams of Fig. 6 may be separate physical components, or logical subdivisions of single physical components, or may be all implemented in an integrated manner in one physical component. The functions of one block shown in the drawings may be divided between multiple components in an implementation, or the functions of multiple blocks shown in the drawings may be combined in single components in an implementation. Hardware components suitable for use in embodiments of the present invention include, but are not limited to, conventional microprocessors, application specific integrated circuits (ASICs), and field-programmable gate arrays (FPGAs). One or more blocks may be implemented as a combination of dedicated hardware to perform some functions and one or more programmed microprocessors and associated circuitry to perform other functions.

A single processor or other unit may fulfil the functions of several items recited in the claims. The mere fact that certain measures are recited in mutually different dependent claims does not indicate that a combination of these measures cannot be used to advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

The flowchart and block diagrams in the Figures illustrate the architecture, functionality, and operation of possible implementations of systems, methods, and computer program products according to various embodiments of the present invention. In this regard, each block in the flowchart or block diagrams may represent a module, segment, or portion of instructions, which comprises one or more executable instructions for implementing the specified logical function(s). In some alternative implementations, the functions noted in the block may occur out of the order noted in the Figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions, the architecture, functionality, and operation of possible implementations of systems, methods, and computer program products according to various embodiments of the present invention.

## Claims

1. A system (100) for calming a subject during breathing difficulties, the system comprising:
a monitor module (110) configured to:
obtain first physiological data comprising at least one first physiological parameter of the subject indicative of a breathing difficulty of the subject;
a process module (120) configured to:
generate, based on the obtained first physiological data, at least one first audio signal comprising white noise and a context sound; and
an audio module (130) configured to:
output the at least one generated first audio signal.

2. The system of claim 1, wherein the context sound comprises at least one of: a medical instruction; a comfort word; a whisper; a whispered instruction; a whispered comfort word; and a personalized message.

3. The system of claim 1 or 2, wherein the context sound is at least partially synthesized and comprises at least one of: a whisper; a whispered instruction; and a whispered comfort word.

4. The system of any of claims 1 to 3, wherein the semantic content of the context sound is associated with a stage in a clinical treatment phase of the subject.

5. The system of any of claims 1 to 4, wherein the process module (120) is further configured to:
analyze the obtained first physiological data to determine at least one of: an anxiety breathlessness score; a bilevel positive airway pressure (BiPAP) tolerance score; and an indication of intubation;
wherein generating the at least one first audio signal is based on the at least one of the anxiety breathlessness score; the BiPAP tolerance score; and the indication of intubation indicating a presence of breathing difficulties of the subject.

6. The system of any of claims 1 to 5, wherein after outputting the at least one generated first audio signal, the monitor module (110) is configured to obtain second physiological data comprising at least one second physiological parameter of the subject indicative of a breathing difficulty of the subject.

7. The system of claim 6, wherein, if the first physiological data and the second physiological data indicates that the subject's breathing difficulty is stable or improving, the process module (120) is configured to:
generate at least one second audio signal comprising the same white noise as the first audio signal and a different context sound to the first audio signal.

8. The system of claim 6 or 7, wherein, if the first physiological data and the second physiological data indicates that the subject's breathing difficulty is getting worse, the process module (120) is configured to:
generate at least one second audio signal comprising at least one of:
a different white noise to the first audio signal and the same context sound as the first audio signal;
the same white noise as the first audio signal and a different context sound to the first audio signal; and
a different white noise and a different context sound to the first audio signal.

9. The system of claim 7 or 8, wherein the at least one second physiological parameter comprises the same parameter as the at least one first physiological parameter, and an indication that the subject's breathing difficulty is stable, improving or getting worse is based on a change between the at least one second and at least one first physiological parameter.

10. The system of any of claims 6 to 9, wherein the system further comprises a feedback module (340) configured to:
determine a clinical trend based on the first physiological data and the second physiological data;
determine a calming effectiveness score of the at least one first audio signal based on the determined clinical trend; and
generate a display control signal describing the clinical trend and the calming effectiveness score.

11. The system of claim 10, wherein the feedback module (340) is further configured to receive user input; and wherein the process module (120) is further configured to generate at least one further audio signal based on the received user input.

12. The system of any of claims 1 to 11, wherein the first physiological data comprises at least one physiological parameter of the subject describing a stress level of the subject and at least one physiological parameter of the subject describing respiration of the subject.

13. The system of any of claims 1 to 12, wherein the at least one first physiological parameter comprises at least one of: respiratory rate; blood oxygen level; tidal volume of the subject; minute ventilation; heart rate variability; skin resistance level; subject-ventilator asynchrony; FiO2; oxygen saturation; and work of breathing.

14. A ventilator (400) comprising:
a breathable gas source (410);
a delivery system (420) for delivering a breathable gas from the breathable gas source (410) to a subject, the delivery of the breathable gas being directed to the subject through a respiratory conduit of the ventilator; and
the system (430) of any of claims 1 to 13.

15. A computer program comprising code means for implementing the following method when said program is run on a system according to any of claims 1 to 13:
obtaining first physiological data (510) comprising at least one first physiological parameter of the subject indicative of a breathing difficulty of the subject;
generating, based on the obtained physiological data, at least one first audio signal (520) comprising white noise and a context sound; and
outputting the at least one generated first audio signal (530).
